# EUROPEAN PATENT SPECIFICATION

(11) **EP 1 534 205 B1**
(45) Date of publication and mention of the grant of the patent: **08.03.2017**
(21) Application number: 03737197.8
(22) Date of filing: 20.06.2003
(51) Int. Cl.: A61F 13/20, A61F 13/26

(54) **TAPERED TAMPON APPLICATOR**
VERENGTER TAMPONAPPLIKATOR
APPLICATEUR DE TAMPON CONIQUE

(30) Priority: 21.06.2002 US 390751 P
(43) Date of publication of application: 01.06.2005
(73) Proprietor: Edgewell Personal Care Brands, LLC, Chesterfield, MO 63017 (US)
(72) Inventor: LEMAY, Jessica, E., New Castle, DE 19720 (US); BENNETT, Kathryn, G., Fairfield, CT 06430 (US); EDGETT, Keith, Middletown, DE 19709 (US); JACKSON, Dane, R., Gilbertsville, PA 19525 (US); RELAI, Jamshid, Dover, DE 19904 (US)
(74) Representative: dompatent von Kreisler Selting Werner - Partnerschaft von Patent- und Rechtsanwälten mbB
(86) International application number: PCT/US2003/019420
(87) International publication number: WO 2004/000160

(56) References cited:
- FR-A- 1 515 087
- US-A- 4 453 925
- US-A- 5 279 541
- US-A- 5 792 096
- US-A- 6 019 743
- US-A- 6 045 526
- US-B1- 6 179 802
- US-S- D 250 663

## Description

### BACKGROUND OF THE INVENTION

### 1. Field of the Invehtion

The present invention relates to an insertion device, such as a catamenial tampon applicator. More particularly, the present invention relates to a tapered tampon applicator. The tapered tampon applicator can be formed from any suitable material, including cardboard. Reference is made to US 5 792 096, FR 1 515 087 and US 6 019 743.

### 2. Description of the Prior Art

The majority of commercial tampon applicators are of approximately uniform cross-section and are formed from two components, namely a barrel and a plunger. The barrel has an insertion end that may be blunt and open-ended. In the alternative, the insertion end of the barrel may be rounded or dome-shaped, and may include a number of petals. This arrangement lends itself to greater ease of insertion and insertion comfort to a user, as opposed to the blunt, open-ended design.

Despite the options of petal-tipped applicators commercially available at present, tampon users are generally dissatisfied with the level of comfort provided by these applicators upon insertion. Through both qualitative and quantitative consumer research, it has been determined that providing a tapered insertion tip greatly enhances the actual and perceived level of comfort associated with inserting a tampon applicator.

An applicator barrel with a rounded or dome-shaped end with petals and associated methods of making such applicators is well known in the art of tampons. However, further modification to the applicator insertion end, such as tapering, especially with cardboard applicators, is not prevalent in the art. This is primarily due to a need to modify existing processes or provide new processes for forming tampons with tapered ends. This can be especially difficult with cardboard applicators.

Therefore, there is a need for a tampon applicator, and more particularly a cardboard applicator, that can be manufactured with a tapered, or non-hemispherical portion or portions on the barrel, which imparts enhanced user comfort and ease of use.

### SUMMARY OF THE INVENTION

It is an object of the present invention to provide a tampon applicator that has a tapered insertion end.

It is another object of the present invention to provide a tampon applicator that has a tapered main barrel body section and a tapered insertion end.

It is still another object of the present invention to provide a tampon applicator that has a tapered insertion end, wherein the cross-section of the tip is not comprised of any straight sections.

It is yet another object of the present invention to provide such a tapered tampon applicator having an insertion end with two or more petals.

It is a further object of the present invention to provide such a tapered tampon applicator having petals with a uniform or substantially uniform thickness.

It is still a further object of the present invention to provide such a tapered tampon applicator having one or more weakened regions formed at the base region of each petal.

It is yet a further object of the present invention to provide such a tapered tampon applicator with a fingergrip area.

It is another object of the present invention to provide such a tapered tampon applicator that has a barrel formed from cardboard.

These and other objects of the present invention will be appreciated from a tampon applicator according to claim 1. Individual embodiments of the invention are the subject matter of the dependent claims.

The tampon applicator has a plunger and a tapered insertion tip, tapered barrel, or both, which provides enhanced insertion comfort to a user. The taper of the barrel is defined by a ratio of the largest radius to the radius of the barrel at the base of the insertion tip. The taper of the insertion tip is defined by the ratio of the length of the taper projection along the vertical axis of the barrel to the projection length along the horizontal axis of the barrel. The insertion end of the barrel preferably has two or more petals for enhanced user comfort. Preferably, each of the petals has a uniform or substantially uniform thickness. The barrel may also include a fingergrip area for ease of use of the applicator during insertion, expulsion of the pledget, and subsequent removal of the applicator from the vagina.

### BRIER DESCRIPTION OF THE DRAWINGS

Fig. 1 is a plan view of a prior art tampon applicator with a rounded or dome-shaped insertion end;
Fig. 2 is a plan view of a tapered tampon applicator insertion end according to the present invention;
Fig. 3 is a plan view of a tapered tampon applicator according to one embodiment of the present invention;
Fig. 4 is a plan view of a tapered tampon applicator according to another embodiment of the present invention;
Fig. 5 is a plan view of the tapered tampon applicator of Fig. 3 with a petal-tip insertion end;
Fig. 6 is a plan view of the tapered tampon applicator of Fig. 4 with a petal-tip insertion end;
Fig. 7 is a plan view of the tapered tampon applicator of Fig. 5 with a weakened region at the base of the insertion end; and
Fig. 8 is a plan view of the tapered tampon applicator of Fig. 6 with a weakened region at the base of the insertion end.

### DETAILED DESCRIPTION OF THE INVENTION

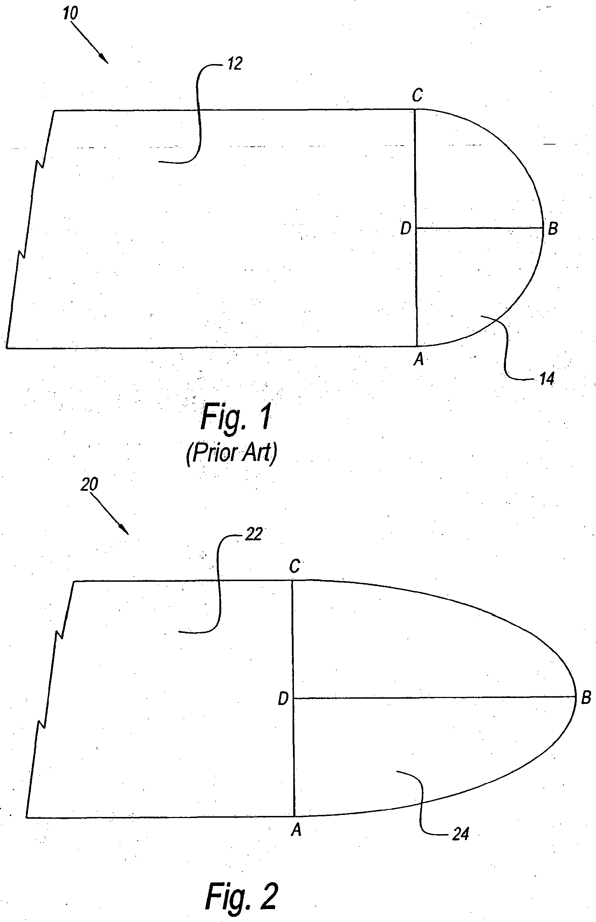

Referring to the drawings and, in particular Fig. 1 , a typical, prior art commercial tampon applicator is represented generally by reference numeral 10. Applicator 10 has a barrel 12 with a rounded or dome-shaped insertion tip 14. Rounded or dome-shaped insertion end 14 can be defined by a ratio of the length of the rounded curve or dome projection along the horizontal axis, as represented by line BD to the projection length along the vertical axis, as represented by line CD. For the rounded or spherical shape of insertion tip 14, length BD/length CD = 1.00.

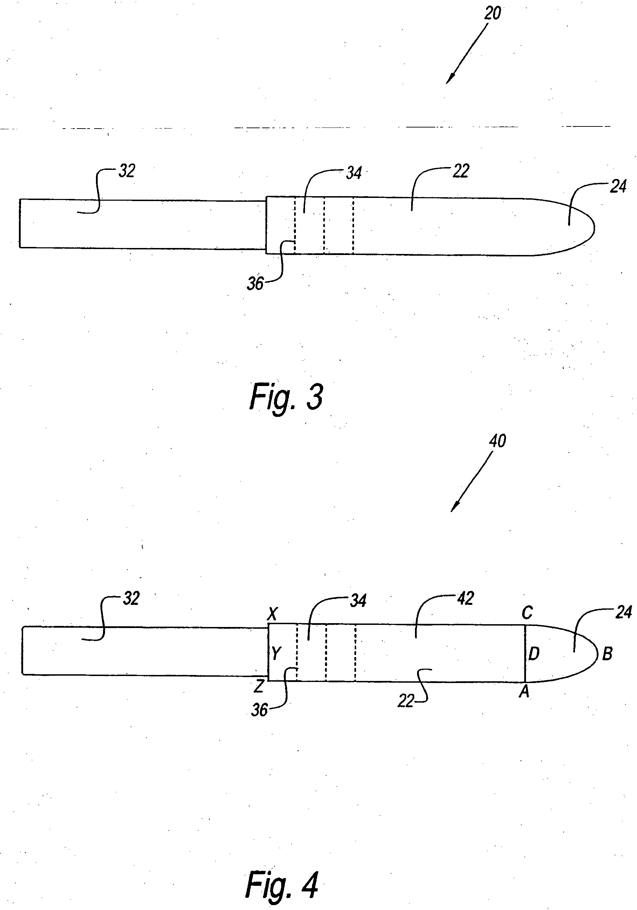

Referring to Figs. 2 and 3, one embodiment of the tampon applicator of the present invention is represented generally by reference numeral 20.

Applicator 20 has a barrel 22 and a tapered insertion tip 24. The more gradual taper of the present invention, as opposed to the rounded or dome-shaped tip of the prior art, affords a user increased comfort, especially during insertion of applicator 20 into the vagina, by gradually parting the vulva-vaginal channel over the longer, tapered insertion tip 24. To achieve such enhanced properties, the taper ratio, represented by the ratio of the length BD of the taper projection along a longitudinal, centerline, or horizontal axis of the barrel to the projection length along a radius or vertical axis CD, is greater than 1 to about 8. Preferably, the taper ratio is about 1.35 to about 5, and more preferably about 1.4 to about 2.3. To enhance the ease of use, barrel 22 may also have a fingergrip area 34. While the shape of the line BC may be substantially straight, approximate a curve, or a combination of both, it is preferred that there is no straight section at any point along the line BC.

Through both quantitative and qualitative consumer testing, it has been determined that an applicator with a tapered tip having a ratio according to the present invention is greatly preferred to current domed-tipped applicators. Tests were performed in which an applicator with a tapered insertion tip having a ratio of approximately 1.7 and a commercial product having a domed insertion tip with a ratio of 1 , as described above, were used separately. The applicator having a tapered insertion tip rated significantly higher at 95% confidence on insertion comfort and appearance of the applicator, and the belief that the applicator would make the product comfortable to use rated higher at 90% confidence. In qualitative consumer work, consumers selected the tampon of the present invention 3 to 1 over domed-tip tampon applicators because they believed the tapered insertion tips would make the applicator more comfortable to insert.

Based on this consumer data it is determined that a tapered insertion tip with a ratio in the range of the present invention provides consumer-meaningful benefits surrounding insertion comfort both in function and perception.

Referring to Fig. 4, another embodiment of the tapered applicator of the present invention is represented generally by reference numeral 40. Applicator 40 has plunger 32 and barrel 22. Barrel 22 has tapered insertion tip 24, and notably tapered barrel section 42. By extending the taper from insertion tip 24 to tapered barrel section 42, user comfort is further enhanced during use by more gradually parting the vulva-vaginal channel over the increased length of tapered barrel section 42 and tapered tip 24, as opposed to just tapered insertion tip 24. To achieve this improved user comfort, the taper ratio of the barrel represented by the ratio of the largest radius XY to the radius CD of the barrel at the base of the insertion tip, is about 1.2 to about 8, and preferably about 1.25 to about 2. The taper ratio of the insertion tip represented by the ratio of the length of the taper projection along the horizontal axis of the barrel BD to the projection length along the vertical axis CD is about 1.3 to about 5, preferably about 1.4 to about 2.3, and more preferably about 1.45 to about 1.75. The angle that the tangent of surface BX at point C creates with AC may be the same or smaller than the angle that the tangent of surface BX at point X creates with XZ. The shape of curves CX and AZ may be substantially straight or they may approximate a curve, or any combinations thereof.

To enhance ease of use, barrel 22 may also have fingergrip area 34. Referring to Figs. 3 and 4, fingergrip area 34 may be formed with any number and/or configuration of gripping structures 36, to further enhance the applicator's grippability. Fingergrip area 34 may be smooth or, more preferably, may include one or more patterned or textured structures extending above and/or below the surface of the fingergrip area.

Suitable gripping structures 36 may include, for example, one or more abrasive materials, embossments, grooves, high wet coefficient of friction materials, lances, pressure sensitive adhesives, protuberances, slits, treads, or any combinations thereof. In addition, gripping structures 36 may be formed in any shape, including, but not limited to, arc, circle, concave, cone, convex, diamond, line, pentagon, hexagon, octagon, oval, polygon, rectangle, rib, square, triangle, or any combinations thereof.

It should be understood that gripping structures 36 may be arranged circumferentially around fingergrip area 34 in any pattern suitable for forming a gripping area. For example, gripping structures 36 can form a distinct pattern, such as, rows or columns, or may be formed intermittently with breaks in structure or in any random order or pattern.

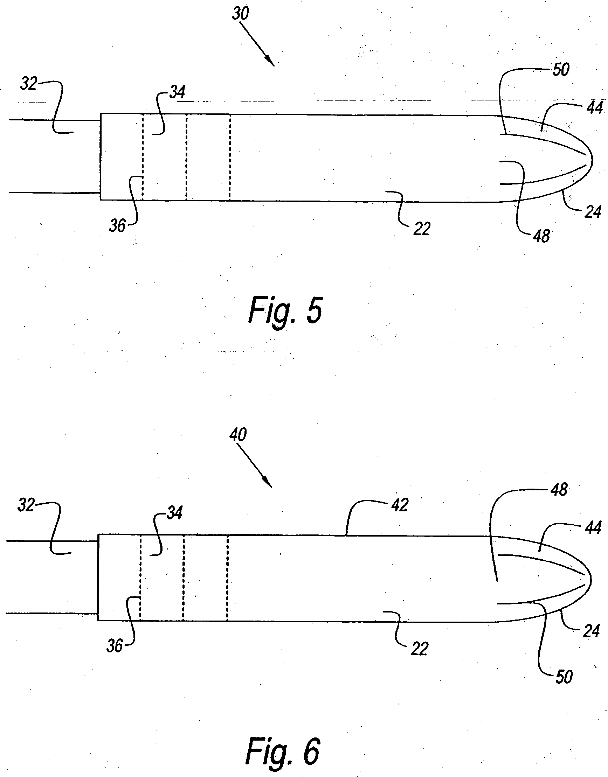

Referring to Figs. 5 and 6, applicators 30, 40, respectively, are depicted with petals 44 formed on insertion tip 24. Insertion tip 24 has about 2 to about 12 individual petals 44, which are curved inwardly at their base region 48. Preferably, insertion tip 24 has about 2 to about 8 individual petals 44, and more preferably about 3 to about 8 individual petals. Each adjacent pair of petals 44 is separated by a radial slit 50.

Petal thickness plays an important role in two key factors of tampon performance, namely, ejection force and petal tip stability. Ejection force is the amount of force the user applies to the plunger to eject the pledget from the applicator. It is desired that this force be low to facilitate easy insertion of the pledget into the body. Petal tip stability is the tendency of the applicator petals to hold their desired shape when subjected to outside factors such as heat, moisture, and any movement that may occur during shipping and/or during insertion into the vagina. If the petal tip is unstable, the petals may "open" or collapse, releasing their desired shape, which may render the tampon uncomfortable to use or even unusable.

The thickness of the applicator petals can be altered to control and/or influence the ejection force and/or petal stability of the applicator. Thin petals, especially those below about 0.56 mm (0.022 inches) in thickness, tend to be lower in ejection force, and decreasing petal thickness may further lower ejection force. However, if the applicator material is not stiff or rigid enough, thin petals may experience tip stability problems, or may collapse inward upon insertion. In this instance, it is helpful to have a pledget whose shape closely approximates or contours to the desired shape of the tapered applicator tip, and/or is compact or dense enough to prevent the petals from collapsing.

According to the present invention, the petals on the tapered tip applicator have a petal thickness of about 0.10 mm (0.004 inches) to about 0.56 mm (0.022 inches). Preferably, the petal thickness is about 0.20 mm (0.008 inches) to about 0.46 mm (0.018 inches), and more preferably, about 0.23 mm (0.009 inches) to about 0.33 mm (0.013 inches).

Increasing the petal thickness to greater than about 0.64 mm (0.025 inches) can help increase the petal stability and/or collapse issue, however this increases ejection force. If the applicator material is such that the petals have to be so thick to provide petal stability that the ejection force becomes higher than acceptable, it is contemplated that a weakened region, as discussed below, could be provided to decrease ejection force.

According to the present invention, the tapered applicator may have petals with a petal thickness of about 0.64 mm (0.025 inches) to about 1.27 mm (0.05 inches). Preferably, the petals may have a thickness of about 0.64 mm (0.025 inches) to about 0.90 mm (0.035 inches), and more preferably about 0.64 mm (0.025 inches) to about 0.76 mm (0.03 inches).

If there is the balance of both ejection force and petal tip stability, there is allowed more flexibility in the pledget design. The pledget may be either rigid or amorphous, and may or may not approximate the shape of the applicator tip, as the tip may not need to be supported by the pledget.

Another important aspect of the petals is petal thickness uniformity. Petal thickness uniformity across the entire area of each petal is advantageous for several reasons. First, it can result in processing efficiencies when making the applicator. Secondly, a uniform thickness ensures that each petal will function properly both during storage and shipment of the applicator, and more importantly during use by a woman. In addition, the uniform petals may be more aesthetically pleasing to the consumer.

In accordance with the present invention, each petal is uniform or substantially uniform in thickness, in that the thickness measured at any point on a given petal does not vary more than about 10% across the entire area of the petal. More preferably, the thickness does not vary more than about 2% across the entire area of the petal.

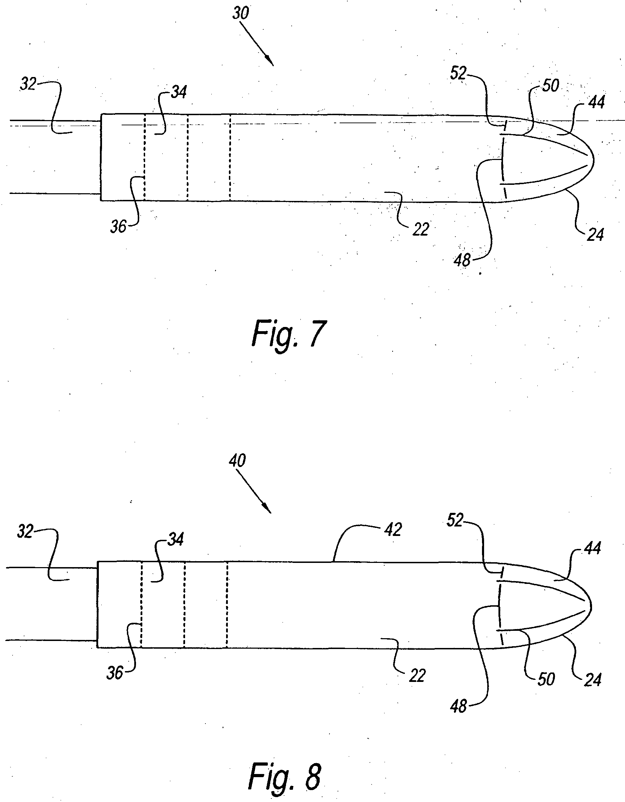

Referring to Fig. 7, in one embodiment of the present invention, one or more circumferential grooves 52 are preferably provided around the inside surface, outside surface, or both of barrel 22 at the petal base region 48 to aid in the curved inward folding of petals 44. Grooves 52 may be formed by any means known in the art. Preferably, grooves 52 are formed by scoring, etching, slitting, molding, or any combinations thereof. Grooves 52 provide a hinge point or weakened region to facilitate bending of petals 44 to their tapered configuration. In addition, the weakened region reduces the expulsion force necessary to eject an absorbent pledget from applicator 30, thus enhancing the ease of use of the applicator.

Referring to Fig. 8, in another embodiment of the present invention, radial slits 50 between the sides of each adjacent pair of petals 44 extend below base region 48, which is the area at the base of the petals, and circumferential groove 52. Advantageously, this allows for tolerances with respect to anticipated manufacturing variations in the location of grooves 52. This also reduces the expulsion force necessary to eject an absorbent pledget from applicator 40, thus enhancing the ease of use of the applicator.

In the case of any tapered tampon applicator of the present invention having a barrel with a maximum outside diameter less than 0.598 inches, the ratio of the extension of radial slits 50 beyond the centerline of the lowest set 60 (relative to the applicator tip) of grooves 52 to the maximum barrel outside diameter is about 0.002 to about 1 and preferably about 0.167 to about 0.5. In addition, the ratio of the width of radial slits 50 at the location of the centerline at the lowest set 60 (relative to the applicator tip) of grooves 52 to the maximum barrel outside diameter is about 0.002 to about 0.25 and preferably about 0.017 to about 0.1.

In the case of any tapered tampon applicator of the present invention having a barrel with a maximum outside diameter greater than or equal to 15.2 mm (0.598 inches) but less than 16.71 mm (0.658 inches), the ratio of the extension of radial slits 50 beyond the centerline of the lowest set 60 (relative to the applicator tip) of grooves 52 to the maximum outside diameter of barrel 22 is about 0.002 to about 0.669 and preferably about 0.152 to about 0.334. The ratio of the width of radial slits 50 at the location of the centerline of the lowest set 60 (relative to the applicator tip) of grooves 52 to the maximum outside diameter of barrel 22 is about 0.002 to about 0.167 and preferably about 0.015 to about 0.067.

In the case of any tapered tampon applicator of the present invention having a barrel with a maximum outside diameter greater than or equal to 16.71 mm (0.658 inches), the extension of radial slits 50 beyond the centerline or the lowest set 60 (relative to the applicator tip) of grooves to the maximum outside diameter of barrel 22 is about 0.001 to about 0.608 and preferably about 0.125 to about 0.304. The width of radial slits 50 at the location of the centerline of the lowest set (relative to the applicator tip) of grooves 52 to the maximum outside diameter of barrel 22 is about 0.001 to about 0.152 and preferably about 0.013 to about 0.061.

In another embodiment of the present invention, grooves 52 may be omitted with the requisite reduction in expulsion force being obtained by the appropriate characteristics of cardboard in conjunction with the initial bending of petals 44 at their base region 48, in accordance with the invention set forth in U.S.-A-5,389,067 and US-A-6,024,716.

Barrel 22 and/or plunger 32 of the present invention may be formed from any suitable material. Suitable materials for forming barrel 22 and/or plunger 32 include, for example, biopolymer, cardboard, cardboard laminate, heat shrink plastic, paper, paper laminate, paper slurry, plastic, plastic tubing, pulp slurry, pulp-molded paper, or any combinations thereof.

Barrel 22 and/or plunger 32 may be internally and/or externally coated with any suitable material to enhance strength and/or reduce surface friction. Suitable coatings include, for example, cellophane, cellulose, epoxy, lacquer, nitrocellulose, nylon, plastic, polyester, polylactide, polyolefin, polyvinyl alcohol, polyvinyl chloride, silicone, wax, or any combinations thereof.

Although it might be implied that the cross-sectional shape of plunger 32 and barrel 22 is circular, due to the use of the terms 'diameter' and 'radius', it should be understood that the cross-sectional shape can be non-circular, such as oval or polygonal. Furthermore, the cross-sectional shape can vary along the length of both plunger 32 and barrel 22. For example, a circular plunger with a- olygonal fingergrip and an oval main body may be formed.

## Claims

1. A tampon applicator comprising a barrel, said barrel having a tapered insertion end with a plurality of petals, wherein the insertion end has a taper ratio greater than 1 to about 8, whereby the taper ratio is represented by the ratio of the length of the taper projection along a longitudinal, centerline or horizontal axis (BD) of the barrel to the projection length along a radius or vertical axis (CD),
- wherein the taper ratio is a length of a projection of the insertion end taper along a longitudinal axis of the barrel to a length of a projection of the insertion end taper along a radius of the barrel at a base region of the plurality of petals; and
- wherein said plurality of petals each have a thickness of about 0.10 mm (0.004 inches) to about 0.55 mm (0.022 inches),
**characterized in that**
- said plurality of petals have a substantially uniform thickness,
- wherein said uniform thickness of each of said plurality of petals varies no more than about 10% across an entire area of said petals

2. The applicator of claim 1, wherein said taper ratio is about 1.3 to about 5.

3. The applicator of claim 1, wherein said taper ratio is about 1.4 to about 2.3.

4. The applicator of claim 1, wherein said taper ratio is about 1.45 to about 1.75.

5. The applicator of claim 1, wherein said plurality of petals each have a thickness of about 0.2 mm (0.008 inches) to about 0.45 mm (0.018 inches).

6. The applicator of claim 1, wherein said plurality of petals each have a thickness of about 0.22 mm (0.009 inches) to about 0.33 mm (0.013 inches).

7. The applicator of claim 1, wherein said uniform thickness of each of said plurality of petals varies no more than about 2% across an entire area of said petals.

8. The applicator of claim 1, wherein said barrel has a barrel taper ratio represented by a ratio of a largest radius of the barrel to a radius of the barrel at a base of the insertion tip of about 1.2 to about 8.

9. The applicator of claim 8, wherein said barrel taper ratio is about 1.25 to about 2.

10. The applicator of claim 1, wherein said barrel has a fingergrip.

11. The applicator of claim 10, wherein said fingergrip has at least one gripping structure.

12. The applicator of claim 11, wherein said at least one gripping structure is circumferentially disposed around said fingergrip.

13. The applicator of claim 11, wherein said at least one gripping structure is selected from the group consisting of one or more abrasive materials, embossments, grooves, high wet coefficient of friction materials, lances, pressure sensitive adhesives, protuberances, slits, treads, and any combinations thereof.

14. The applicator of claim 11, wherein said at least one gripping structure is formed in a shape selected from the group consisting of arc, circle, concave, cone, convex, diamond, hexagon, line, octagon, oval, pentagon, polygon, rectangle, rib, square, triangle, and any combinations thereof.

15. The applicator of claim 11, wherein said at least one gripping structure is raised, depressed, or a combination thereof.

16. The applicator of claim 1, wherein said plurality of petals is about two petals to about eight petals.

17. The applicator of claim 16, wherein each adjacent pair of said about two petals to about eight petals is separated by a radial slit.

18. The applicator of claim 17, wherein said radial slit extends below a base region of said about two petals to about eight petals.

19. The applicator of claim 18, wherein said base region has at least one circumferential groove.

20. The applicator of claim 19, wherein said barrel has a maximum outside diameter less than 15.2 mm (0.598 inches).

21. The applicator of claim 20, further comprising a ratio of an extension of said radial slit beyond said groove to said maximum outside diameter of said barrel of about 0.002 to about 1.

22. The applicator of claim 20, further comprising a ratio of a width of said radial slit at said groove to said maximum outside diameter of said barrel of about 0.002 to about 0.25.

23. The applicator of claim 19, wherein said barrel has a maximum outside diameter of greater than or equal to 15.2 mm (0.598 inches) to less than 16.7 mm (0.658 inches).

24. The applicator of claim 23, further comprising a ratio of an extension of said radial slit beyond said groove to said maximum outside diameter of said barrel of about 0.002 to about 0.669.

25. The applicator of claim 23, further comprising a ratio of a width of said radial slit at said groove to said maximum outside diameter of said barrel of about 0.002 to about 0.167.

26. The applicator of claim 19, wherein said barrel has a maximum outside diameter greater than or equal to 16.7 mm (0.658 inches).

27. The applicator of claim 26, further comprising a ratio of an extension of said radial slit beyond said groove to said maximum outside diameter of said barrel of about 0.001 to about 0.608.

28. The applicator of claim 26, further comprising a ratio of a width of said radial slit at said groove to said maximum outside diameter of said barrel of about 0.001 to about 0.152.

29. The applicator of claim 16, wherein said about two to about eight petals have a weakened base region formed by bending said petals back and forth, thereby breaking one or more bonds.

30. The applicator of claim 1, wherein said barrel is formed from a material selected from the group consisting of biopolymer, cardboard, cardboard laminate, heat shrink plastic, paper, paper slurry, paper laminate, plastic, plastic tubing, pulp slurry, pulp-molded paper, and any combinations thereof.

31. The applicator of claim 1, wherein said barrel is formed from cardboard.

32. The applicator of claim 1, wherein said barrel has a surface that is coated with a material selected from the group consisting of cellophane, cellulose, epoxy, lacquer, nitrocellulose, nylon, plastic, polyester, polylactide, polyolefin, polyvinyl alcohol, polyvinyl chloride, silicone, wax, and any combinations thereof.

33. The applicator of claim 32, wherein said surface is selected from the group consisting of an outer surface, an inner surface, and any combination thereof.

## Patentansprüche

1. Tamponapplikator mit einem Röhrchen, wobei das Röhrchen ein sich verjüngendes Einführende mit mehreren blütenblattartig aufgehenden Segmenten aufweist, wobei das Einführende ein Verjüngungsverhältnis größer als 1 bis ungefähr 8 aufweist, wobei das Verjüngungsverhältnis durch das Verhältnis der Länge des sich verjüngenden Vorsprungs entlang einer Längs-, Mittel- oder horizontalen Achse (BD) des Röhrchens zu der Vorsprunglänge entlang eines Radius oder einer vertikalen Achse (CD) wiedergegeben ist,
- wobei das Verjüngungsverhältnis eine Länge eines Vorsprungs der Verjüngung des Einführendes entlang einer Längsachse des Röhrchens zu einer Länge eines Vorsprungs der Verjüngung des Einführendes entlang eines Radius des Röhrchens an einem Basisbereich der mehreren blütenblattartig aufgehenden Segmente angibt; und
- wobei die mehreren blütenblattartig aufgehenden Segmente jeweils eine Dicke von ungefähr 0,10 mm (0,004 Inch) bis ungefähr 0,55 mm (0,022 Inch) aufweisen,
**dadurch gekennzeichnet, dass**
- die mehreren blütenblattartig aufgehenden Segmente eine im Wesentlichen gleichmäßige Dicke aufweisen,
- wobei die gleichförmige Dicke jedes der mehreren blütenblattartig aufgehenden Segmente über die gesamte Fläche der blütenblattartig aufgehenden Segmente um nicht mehr als ungefähr 10% variiert.

2. Applikator nach Anspruch 1, bei welchem das Verjüngungsverhältnis ungefähr 1,3 bis ungefähr 5 beträgt.

3. Applikator nach Anspruch 1, bei welchem das Verjüngungsverhältnis ungefähr 1,4 bis ungefähr 2,3 beträgt.

4. Applikator nach Anspruch 1, bei welchem das Verjüngungsverhältnis ungefähr 1,45 bis ungefähr 1,75 beträgt.

5. Applikator nach Anspruch 1, bei welchem die mehreren blütenblattartig aufgehenden Segmente jeweils eine Dicke von ungefähr 0,2 mm (0,008 Inch) bis ungefähr 0,45 mm (0,018 Inch) aufweisen.

6. Applikator nach Anspruch 1, bei welchem die mehreren blütenblattartig aufgehenden Segmente jeweils eine Dicke von ungefähr 0,22 mm (0,009 Inch) bis ungefähr 0,33 mm (0,013 Inch) aufweisen.

7. Applikator nach Anspruch 1, bei welchem die gleichförmige Dicke jedes der mehreren blütenblattartig aufgehenden Segmente über die gesamte Fläche der blütenblattartig aufgehenden Segmente um nicht mehr als ungefähr 2% variiert.

8. Applikator nach Anspruch 1, bei welchem das Röhrchen ein Röhrchenverjüngungsverhältnis aufweist, das durch ein Verhältnis eines größten Radius des Röhrchens zu einem Radius des Röhrchens an der Basis der Einführspitze von ungefähr 1,2 bis ungefähr 8 wiedergegeben ist.

9. Applikator nach Anspruch 8, bei welchem das Röhrchenverjüngungsverhältnis ungefähr 1,25 bis ungefähr 2 beträgt.

10. Applikator nach Anspruch 1, bei welchem das Röhrchen einen Anfassbereich aufweist.

11. Applikator nach Anspruch 10, bei welchem der Anfassbereich mindestens eine Greifstruktur aufweist.

12. Applikator nach Anspruch 11, bei welchem die mindestens eine Greifstruktur umfangsmäßig um den Anfassbereich angeordnet ist.

13. Applikator nach Anspruch 11, bei welchem die mindestens eine Greifstruktur aus der Gruppe gewählt ist, welche aus einem oder mehreren Abrasivmaterialien, Prägungen, Rillen, Materialien mit hohem Reibungskoeffizienten im nassen Zustand, Rippen, Haftklebern, Vorsprüngen, Schlitzen, Profilen und beliebigen Kombinationen derselben gebildet ist.

14. Applikator nach Anspruch 11, bei welchem die mindestens eine Greifstruktur mit einer Form ausgebildet ist, welche aus der Gruppe ausgewählt ist, welche durch die Formen bogenförmig, kreisförmig, konkav, konisch, konvex, rautenförmig, hexagonal, linienförmig, achteckig, oval, fünfeckig, polygonal, rechteckig, rippenfömig, quadratisch, dreieckig und beliebigen Kombinationen derselben gebildet ist.

15. Applikator nach Anspruch 11, bei welchem die mindestens eine Greifstruktur erhaben, vertieft oder als eine Kombination daraus ausgebildet ist.

16. Applikator nach Anspruch 1, bei welchem die Anzahl der mehreren blütenblattartig aufgehenden Segmente zwischen ungefähr zwei blütenblattartigen Teilen und ungefähr acht blütenblattartig aufgehenden Segmente beträgt.

17. Applikator nach Anspruch 16, bei welchem jedes benachbarte Paar der zwischen ungefähr zwei blütenblattartig aufgehenden Segmente und ungefähr acht blütenblattartig aufgehenden Segmente durch einen radialen Schlitz getrennt ist.

18. Applikator nach Anspruch 17, bei welchem sich der radiale Schlitz unter einen Basisbereich der ungefähr zwei blütenblattartig aufgehenden Segmente bis ungefähr acht blütenblattartig aufgehenden Segmente erstreckt.

19. Applikator nach Anspruch 18, bei welchem der Basisbereich mindestens eine Umfangsnut aufweist.

20. Applikator nach Anspruch 19, bei welchem das Röhrchen einen maximalen Außendurchmesser von weniger als 15,2 mm (0,598 Inch) aufweist.

21. Applikator nach Anspruch 20, ferner mit einem Verhältnis der Erstreckung des radialen Schlitzes über die Nut hinaus zu dem maximalen Außendurchmesser des Röhrchens von ungefähr 0,002 bis ungefähr 1.

22. Applikator nach Anspruch 20, ferner mit einem Verhältnis der Breite des radialen Schlitzes an der Nut zu dem maximalen Außendurchmesser des Röhrchens von ungefähr 0,002 bis ungefähr 0,25.

23. Applikator nach Anspruch 19, bei welchem das Röhrchen einen maximalen Außendurchmesser größer als oder gleich 15,2 mm (0,589 Inch) bis weniger als 16,7 mm (0,658 Inch) aufweist.

24. Applikator nach Anspruch 23, ferner mit einem Verhältnis der Erstreckung des radialen Schlitzes über die Nut hinaus zu dem maximalen Außendurchmesser des Röhrchens von ungefähr 0,002 bis ungefähr 0,669.

25. Applikator nach Anspruch 23, ferner mit einem Verhältnis der Breite des radialen Schlitzes an der Nut zu dem maximalen Außendurchmesser des Röhrchens von ungefähr 0,002 bis ungefähr 0,167

26. Applikator nach Anspruch 19, bei welchem das Röhrchen einen maximalen Außendurchmesser größer als oder gleich 16,7 mm (0,658 Inch) aufweist.

27. Applikator nach Anspruch 26, ferner mit einem Verhältnis der Erstreckung des radialen Schlitzes über die Nut hinaus zu dem maximalen Außendurchmesser des Röhrchens von ungefähr 0,001 bis ungefähr 0,608.

28. Applikator nach Anspruch 26, ferner mit einem Verhältnis der Breite des radialen Schlitzes an der Nut zu dem maximalen Außendurchmesser des Röhrchens von ungefähr 0,001 bis ungefähr 0,152.

29. Applikator nach Anspruch 16, bei welchem die ungefähr zwei blütenblattartig aufgehenden Segmente bis ungefähr acht blütenblattartig aufgehenden Segmente einen geschwächten Basisbereichs aufweisen, der durch Vor- und Zurückbiegen der blütenblattartig aufgehenden Segmente gebildet sind, wodurch eine oder mehrere Verbindungen zerbrochen werden..

30. Applikator nach Anspruch 1, bei welchem das Röhrchen aus einem Material gebildet ist, das aus der Gruppe gewählt ist, welche aus Biopolymer, Pappe, Papplaminat, Wärmeschrumpfkunstoff, Papier, Papierschlamm Papierlaminat, Kunststoff, Kunststoffrohre, Pulpe, Papier aus geformter Pulpe und beliebigen Kombinationen derselben besteht.

31. Applikator nach Anspruch 1, bei welchem das Röhrchen aus Pappe besteht.

32. Applikator nach Anspruch 1, bei welchem das Röhrchen eine Oberfläche aufweist, die mit einem Material beschichtet ist, welches aus der Gruppe ausgewählt ist, welche aus Zellophan, Zellulose, Epoxidharz, Lack, Nitrozellulose, Nylon, Kunststoff, Polyester, Polylactid, Polyolefin, Polyvinylalkohol, Polyvinylchlorid, Silikon, Wachs und beliebigen Kombinationen derselben besteht.

33. Applikator nach Anspruch 32, bei welchem die Oberfläche aus der Gruppe gewählt ist, die aus einer Außenfläche, einer Innenfläche und beliebigen Kombinationen derselben besteht.

## Revendications

1. Applicateur de tampon, comprenant un tube, ledit tube comportant une extrémité d'insertion fuselée pourvue d'une pluralité de pétales, l'extrémité d'insertion ayant un rapport de finesse de plus de 1 à environ 8, le rapport de finesse étant représenté par le rapport entre la longueur de la protubérance fuselée, le long de l'axe longitudinal, central ou horizontal (BD) du tube, et la longueur de la protubérance le long d'un rayon ou axe vertical (CD),
- le rapport de finesse étant le rapport entre une longueur de la protubérance fuselée de l'extrémité d'insertion, le long d'un axe longitudinal du tube, et une longueur de la protubérance fuselée de l'extrémité d'insertion le long d'un rayon du tube au niveau d'une zone de base de la pluralité de pétales ; et
- chaque pétale de ladite pluralité de pétales ayant une épaisseur d'environ 0,10 mm (0,004 pouces) à environ 0,55 mm (0,022 pouces),
**caractérisé en ce que**
- ladite pluralité de pétales a une épaisseur sensiblement uniforme,
- ladite épaisseur uniforme de chaque pétale de ladite pluralité de pétales ne varie pas plus que de 10% environ sur toute une zone desdites pétales.

2. Applicateur selon la revendication 1, dans lequel ledit rapport de finesse est d'environ 1,3 à environ 5.

3. Applicateur selon la revendication 1, dans lequel ledit rapport de finesse est d'environ 1,4 à environ 2,3.

4. Applicateur selon la revendication 1, dans lequel ledit rapport de finesse est d'environ 1,45 à environ 1,75.

5. Applicateur selon la revendication 1, dans lequel chaque pétale de ladite pluralité de pétales a une épaisseur comprise entre environ 0,2 mm (0,008 pouces) et environ 0,45 mm (0,018 pouces).

6. Applicateur selon la revendication 1, dans lequel chaque pétale de ladite pluralité de pétales a une épaisseur comprise entre environ 0,22 mm (0,009 pouces) et environ 0,33 mm (0,013 pouces).

7. Applicateur selon la revendication 1, dans lequel ladite épaisseur uniforme de chaque pétale de ladite pluralité de pétales ne varie pas plus que de 2% environ sur toute une zone desdites pétales.

8. Applicateur selon la revendication 1, dans lequel ledit tube a un rapport de finesse de tube, représenté par un rapport entre le rayon le plus grand du tube et un rayon du tube au niveau d'une base de la pointe d'insertion, compris entre environ 1,2 et environ 8.

9. Applicateur selon la revendication 8, dans lequel ledit rapport de finesse du tube est compris entre environ 1,25 et environ 2.

10. Applicateur selon la revendication 1, dans lequel ledit tube comporte une prise pour les doigts.

11. Applicateur selon la revendication 10, dans lequel ladite prise pour les doigts présente au moins une structure de préhension.

12. Applicateur selon la revendication 11, dans lequel ladite au moins une structure de préhension est disposée circonférentiellement autour de la dite prise pour les doigts.

13. Applicateur selon la revendication 11, dans lequel ladite au moins une structure de préhension est choisie dans le groupe consistant en un ou plusieurs matériaux abrasifs, bossages, rainures, matériaux à coefficient de frottement élevé à l'état humide, lances, adhésifs sensibles à la pression, protubérances, fentes, sculptures, et toutes combinaisons de ceux-ci.

14. Applicateur selon la revendication 11, dans lequel ladite au moins une structure de préhension présente une forme choisie dans le groupe consistant en les formes d'arc, de cercle, concave, conique, convexe, en diamant, hexagonale, linéaire, octogonale, ovale, pentagonale, polygonale, rectangulaire, à nervure, carrée, triangulaire et toutes combinaisons de celles-ci.

15. Applicateur selon la revendication 11, dans lequel ladite au moins une structure de préhension est en relief, en creux ou une combinaison des deux.

16. Applicateur selon la revendication 1, dans lequel ladite pluralité de pétales présente entre environ deux pétales et environ huit pétales.

17. Applicateur selon la revendication 16, dans lequel chaque paire adjacente desdites pétales au nombre de environ deux à environ huit est séparée par une fente radiale.

18. Applicateur selon la revendication 17, dans lequel ladite fente radiale s'étend au-dessous d'une région de base desdites pétales au nombre de environ deux à environ huit.

19. Applicateur selon la revendication 18, dans lequel ladite région de base présente au moins une rainure circonférentielle.

20. Applicateur selon la revendication 19, dans lequel ledit tube a un diamètre extérieur maximum inférieur à 15,2 mm (0,598 pouces).

21. Applicateur selon la revendication 20, comprenant, en outre, un rapport entre une extension de ladite fente radiale au-delà de ladite rainure et ledit diamètre extérieur maximum dudit tube compris entre environ 0,002 et environ 1.

22. Applicateur selon la revendication 20, comprenant, en outre, un rapport entre une largeur de ladite fente radiale au niveau de ladite rainure et ledit diamètre extérieur maximum dudit tube compris entre environ 0,002 et environ 0,25.

23. Applicateur selon la revendication 19, dans lequel ledit tube a un diamètre extérieur maximum supérieur ou égal à 15,2 mm (0,598 pouces) et inférieur à 16,7 mm (0,658 pouces).

24. Applicateur selon la revendication 23, comprenant, en outre, un rapport entre une extension de ladite fente radiale au-delà de ladite rainure et ledit diamètre extérieur maximum dudit tube compris entre environ 0,002 et environ 0,669.

25. Applicateur selon la revendication 23, comprenant, en outre, un rapport entre une largeur de ladite fente radiale au niveau de ladite rainure et ledit diamètre extérieur maximum dudit tube compris entre environ 0,002 et environ 0,167.

26. Applicateur selon la revendication 19, dans lequel ledit tube a un diamètre extérieur maximum supérieur ou égal à 16,7 mm (0,658 pouces).

27. Applicateur selon la revendication 26, comprenant, en outre, un rapport entre une extension de ladite fente radiale au-delà de ladite rainure et ledit diamètre extérieur maximum dudit tube compris entre environ 0,001 et environ 0,608.

28. Applicateur selon la revendication 26, comprenant, en outre, un rapport entre une largeur de ladite fente radiale au niveau de ladite rainure et ledit diamètre extérieur maximum dudit tube compris entre environ 0,001 et environ 0,152.

29. Applicateur selon la revendication 16, dans lequel lesdites pétales au nombre de environ deux à environ huit présentent une région de base affaiblie formée par flexion desdites pétales dans un mouvement de va-et-vient, rompant ainsi une ou plusieurs liaisons.

30. Applicateur selon la revendication 1, dans lequel ledit tube est réalisé dans un matériau choisi dans le groupe consistant en : biopolymère, carton, stratifié de carton, plastique thermorétractable, papier, pâte à papier, stratifié papier, plastique, tubulure de plastique, pâte à papier liquide, pâte à papier moulée, et toutes combinaisons de ceux-ci.

31. Applicateur selon la revendication 1, dans lequel ledit tube est formé de carton.

32. Applicateur selon la revendication 1, dans lequel ledit tube a une surface revêtue d'un matériau choisi dans le groupe consistant en : cellophane, cellulose, époxy, vernis, nitrocellulose, nylon, plastique, polyester, polylactide, polyoléfine, alcool polyvinylique, chlorure de polyvinyle, silicone, cire, et toutes combinaisons de ceux-ci.

33. Applicateur selon la revendication 32, dans lequel ladite surface est choisie dans le groupe consistant en une surface extérieure, une surface intérieure, et toute combinaison des deux.
